# EUROPEAN PATENT APPLICATION

(11) **EP 1 889 619 A1**
(43) Date of publication of application: **20.02.2008**
(21) Application number: 06708873.2
(22) Date of filing: 10.02.2006
(51) Int. Cl.: A61K 31/433, A61P 25/28, C07D 285/08, C07D 417/04

(54) **USE OF THIADIAZOLIDINE-DERIVED COMPOUNDS AS NEUROGENIC AGENTS**

(30) Priority: 10.02.2005 ES 200500345
(71) Applicant: Neuropharma, S.A., 28760 Madrid (ES)
(72) Inventor: MARTÍNEZ GIL, Ana, E-28004 Madrid (ES); DE LUNA MEDINA, Rosario, E-28032 Madrid (ES); PÉREZ CASTILLO, Ana, E-28760 Tres Cantos - Madrid (ES); ALONSO CASCÓN, Mercedes, E-28036 Madrid (ES)
(74) Representative: ABG Patentes, S.L.
(86) International application number: PCT/ES2006/000055
(87) International publication number: WO 2006/084934

(57) **Abstract**

The invention relates to the use of heterocyclic compounds in the preparation of a medicament for regenerating damaged neuronal tissue.

According to a preferred embodiment, formula I compounds are used to produce a medicament for regenerating neuronal tissue in pathologies or conditions that involve neuronal damage or death in the central nervous system or peripheral nervous system.

## Description

### FIELD OF THE INVENTION

The invention relates to the use of heterocyclic compounds in the preparation of a medicament for regenerating damaged neuronal tissue.

### BACKGROUND OF THE INVENTION

The Central Nervous System has a limited capacity to produce new neurons, therefore making it vulnerable to external aggressions and different diseases. In mammals, most neurons are generated in the prenatal period, but it has been shown that neurons continue to be generated, even in adults, in at least two regions of mammals' brains: the dentate gyrus of the hippocampus and the lateral ventricles (Kaplan, M.S. and Hinds J.W. (1977). Science 197, 1092-4). These neurons derive from populations of "stems cells" and their less multipotent progeny, "progenitor cells". Granule neurons arise in the hippocampus in the subgranular zone of the dentate gyrus (SGZ) (Gage, F.H., Kempermann, G., Palmer, T.D., Peterson, D.A. and Ray, J. (1998). J Neurobiol 36, 249-66). Progenitor cells are formed in the front region of the lateral ventricles and migrate to the olfactory bulb via an area called the rostral migratory stream (RMS).

The RMS starts in the most rostral area and ends in the olfactory bulb, where the neural precursors move radially and turn into interneurons known as granule and periglomerular cells (Lois, C. and Alvarez-Buylla, A. (1994). Science 264, 1145-8). Understanding the factors that promote the division of these stem cells and regulate the proliferation, migration, differentiation and survival of their progeny, the most differentiated "progenitor cells", is a very important step towards achieving the ability to use populations of endogenous stem cells to induce the production of new neurons and glia. This ability would be of great use in clinical medicine when trying to replace neural cells lost as a consequence of external damage or diseases. There are two particularly well characterised factors that affect the proliferation of these cells: epidermal growth factor (EGF) and basic fibroblast growth factor (FGF-2) (Cameron, H.A., Hazel, T.G., and McKay, R.D. (1998). J Neurobiol 36, 287-306). These factors promote the formation of "neurospheres", which are undifferentiated spherical cell clusters that can be induced to differentiate neurons, oligodendrocytes or astrocytes when transferred to a substrate such as polylysine (Reynolds, B.A. and Weiss, S. (1992). Science 255, 1707-10). When these neurospheres are dissociated into individual cells and cultured, a small fraction of them continues to form neurospheres. It is thought that neurospheres are made up of at least two different cell types, cells with stem cell characteristics and progenitor cells that divide very rapidly (Morshead, C.M., Reynolds, B.A., Craig, C.G., et al. (1994). Neuron 13, 1071-82).

Compounds called thiadiazolidinones (TDZDs) were initially described as inhibitors of glycogen synthase kinase-3 beta (GSK-3 beta) (Martinez, A., Alonso, M., Castro, A., Perez, C. Moreno, F.J. (2002). J.Med.Chem 45:1292-99). Due to this effect, it has been postulated that they may be potential therapeutic compounds for the treatment of Alzheimer's disease and other neurodegenerative diseases with inflammatory processes (Dorronsoro, I., Castro, A., Martinez, A. (2002). Exp.Opin.Ther.Pat. 12:1539-44). In vivo studies of GSK-3 beta on transgenic mice have confirmed this potential therapeutic effect (Lucas, J.J., Hernandez, F., Gomez-Ramos, P., Moran, M.A., Avila, J. (2001). EMBO J. 20:27-39). Furthermore, recent data show that these compounds are also potent anti-inflammatory and neuroprotective agents in primary cultures of neural cells and that these may be mediated by the peroxisome proliferator-activated receptor-gamma (PPAR-gamma) (EP04077903.5)

Cell death, and also neuronal death, can be placed in two broad groups: necrosis and apoptosis.

The term necrosis covers violent and catastrophic processes, where cell degeneration is passive without requiring energy in the form of ATP. It often occurs as a consequence of injury or exposure to toxins. It includes an acute loss of regulation and loss of cell function that involves excessive osmotic effects and ends in lysis of the cell membrane, releasing the intracellular content. This phenomenon also leads to the death of neighbouring cells, whilst attracting inflammatory cells, which mean that new cells that develop this type of cell death are often found in areas where necrotic cells are observed, as are cells that cause an inflammatory reaction and a fibrous scar that deforms the tissue and organ concerned.

The second type of cell death is known as apoptosis or programmed cell death. In this process the cells self-destruct without triggering inflammatory reactions or leaving scarred tissue. Apoptosis results in a mechanism that disposes of unwanted, damaged or unknown cells, which helps protect against potential diseases.

In a pathology or condition, neuronal death can occur by either of the two mechanisms, or a combination of the two can coexist in the same pathology or condition.

It is widely known that numerous diseases and conditions related to the brain and the Nervous System are associated with neuronal death, including states such as depression, attention deficit or bipolar disorders, neurodegenerative diseases such as Alzheimer's or Parkinson's, and damage to the Nervous System as a result of injuries. Within this context it has been established that the induction of neurogenesis or the promotion of neuronal regeneration is clearly linked to improvements in said patients.

Therefore, in the publication of WO 02/062387A1, the authors have established that the promotion of neuronal regeneration improves the state of patients with acute neuronal injury, such as crush injury, acute stroke, ischaemia, neurotraumatic insult, neurotrauma or spinal cord injury.

Studies carried out by researchers at Yale University ("*Sustained Use of Anti-depressants Increases Cell Growth and Protects Cells in the Brain, Sciencedaily 2000-12-15"*) describe the fact that different states of depression are linked to neuronal death. Moreover, examination of images of the brain in patients with depression or after post-traumatic stress disorder has shown that there is a reduction in the volume of the hippocampus, which is linked to a neuronal atrophy or even a loss thereof. The same researchers have proved that the use of anti-depressant agents leads to an increase in neurons in the hippocampus of adults.

What is more, the document *"*Increasing hippocampal neurogenesis: a novel mechanism for antidepressant drugs. Malberg, J., Schechter, L.,Curr. Pharm. Desing 2005 11 (2) 145-55" describes how treatment with anti-depressant drugs boosts or increases neurogenesis in the hippocampus in pathological syndromes linked to states of depression. According to another document, *"*Chronic antidepressant treatment increases neurogenesis in adult rat hippocampus. J. Neurosci. 2000 20 (24) 9104-10*",* recent studies have suggested that atrophy induced by stress and neuron loss in the hippocampus can contribute to the physiopathology of stress, and they show the positive effect of anti-depressants in neurogenesis of the hippocampus. On the other hand, the document *"*Mechanisms of action of antidepressants: from neurotransmitter systems to signaling pathways. Taylor, C., Fricker, A.D., Devi, L.A. Gomes, I. Cell Signal. 2005 17 (5) 549-57*."* describes the different neurotransmitter systems, which are affected by states of depression and anxiety, and how these systems are modulated by treatment with anti-depressants in order to direct them towards molecules and signalling pathways, which are activated during the neurogenesis induced by the receptors of said neurotransmitters.

There is therefore a great deal of interest in finding compounds that have neurogenic properties and are therefore helpful in preventing or treating the aforementioned diseases or conditions. The compounds must also have good properties "as a drug", i.e. acceptable pharmaceutical properties in terms of their administration, distribution, metabolism and excretion.

### SUMMARY OF THE INVENTION

Surprisingly, it has been found that compounds of the thidiazolidinone (TDZD) type such as those disclosed in applications WO 0185685, US2003/0195238 and EP04075997.9 present neurogenesis activity and are therefore useful in the preparation of a medicament for regenerating damaged neuronal tissue.

According to a first essential aspect, the present invention is aimed at the use of a general formula (I) compound: 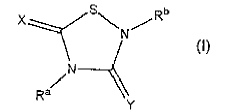 or a pharmaceutically acceptable salt, prodrug or solvate thereof, wherein:
- R^{a} and R^{b}: are each independently selected from hydrogen, substituted or unsubstituted alkyl, substituted or unsubstituted cycloalkyl, haloalkyl, substituted or unsubstituted aryl, substituted or unsubstituted -(Z)ₙ-aryl, substituted or unsubstituted heteroaryl, -OR⁵, -C(O)R⁵, -C(O)OR⁵, -(Z)ₙC(O)OR⁵ and -S(O)ₜ-R⁷;
- Z: is independently selected from -C(R³)(R⁴)-, -C(O)-, -O-, -C(=NR⁵)-, -S(O)ₜ-R⁷ and N(R⁵)-;
- n: is zero, one or two;
- t: is zero, one or two;
- R³ and R⁴: are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, COR⁷, -C(O)OR⁷, -C(O)NR⁷R⁸ -C=NR⁷, -CN, -OR⁷, -OC(O)R⁷, -S(O)ₜ-R⁷, -NR⁷R⁸, -NR⁷C(O)R⁸, -NO -NO₂, -N=CR⁷R⁸ or halogen, wherein R³ and R⁴ together can form a =O group;
- X and Y: are each independently selected from =O, =S, =N(P⁵) and =C (R¹) (R²);
- R¹ and R²: are each independently selected from hydrogen, substituted or unsubstituted alkyl and substituted or unsubstituted cycloalkyl;
- R⁵: is selected from hydrogen, substituted or unsubstituted alkyl, substituted or unsubstituted aryl and substituted or unsubstituted heterocyclyl;
- R⁷ and R⁸: are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy and halogen
in the preparation of a medicament for regenerating damaged neuronal tissue.
According to a preferred embodiment, the formula (I) compound that is used presents formula (II): 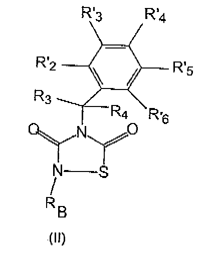 wherein:
R_{B} is selected from substituted or unsubstituted alkyl, substituted or unsubstituted cycloalkyl, haloalkyl, substituted or unsubstituted aryl, substituted or unsubstituted aralkyl, substituted or unsubstituted alkylaryl, substituted or unsubstituted heteroaryl, -OR⁵ and -S(O)ₜ-R⁷;
R³, R⁴, R²', R³', R⁴', R⁵' and R⁶' are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, COR⁷, -C(O)OR⁷, -C(O)NR⁷R⁸ -C=NR⁷, -CN, -OR⁷, OC(O)R⁷, -S(O)ₜ-R⁷, -N R⁷R⁸, -NR⁷C(O)R⁸, -NO₂, -N=CR⁷R⁸ or halogen, wherein R³ and R⁴ together can form a =O group, and wherein any pair of R³R²', R³R⁶', R⁴R²', R⁴R⁶', R²'R³', R³'R⁴', R⁴'R⁵', R⁵'R⁶' or R⁷R⁸ together can form a cyclic substituent; t is 0, 1, 2, 3
R⁵ is selected from hydrogen, alkyl, aryl and heterocyclyl;
R⁷ and R⁸ are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy and halogen.
According to a preferred embodiment, formula I compounds are used to produce the medicament for regenerating neuronal tissue in pathologies and conditions that involve neuronal damage or death in the central nervous system or peripheral nervous system.

### BRIEF DESCRIPTION OF THE FIGURES

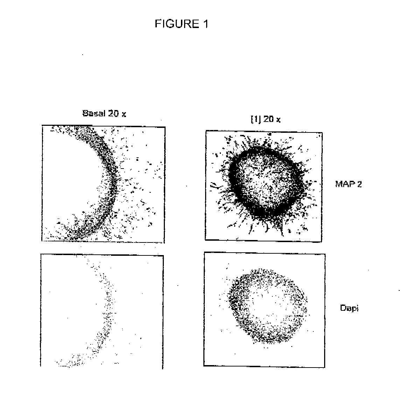

- **Figures 1, 3 and 5**: show the astrocyte differentiation of cells present in isolated neurospheres of the cerebral cortex and hippocampus of 2-day-old neonatal rats due to the addition of formula (I) compounds [1] - [4]. "Basal" refers to neurospheres grown in the absence of formula (I) compound; "x" indicates the number of increases; "DAPI" refers to fluorescent staining by 4' ,6-diamidino-2-phenylindole (DAPI); GFAP refers to viewing by GFAP (glial fibrillary acidic protein) staining; "Nestin" refers to staining by nestin, which is present in immature neural cells; "Mixture" refers to staining by both nestin and GFAP.

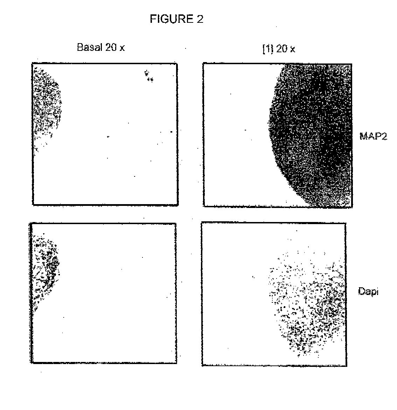

- **Figures 2, 4 and 6**: show the neuronal differentiation of cells present in isolated neurospheres of the cerebral cortex and hippocampus of 2-day-old neonatal rats due to the addition of formula (I) compounds [1]-[4]; MAP-2 refers to viewing by fluorescent staining by MAP-2 (microtubule-associated protein 2); "DAPI" refers to fluorescent staining by 4',6-diamidino-2-phenylindole (DAPI); "Nestin" refers to staining by nestin, which is present in immature neural cells; "Mixture" refers to staining by both nestin and MAP-2.

### DETAILED DESCRIPTION OF THE INVENTION

It has now been found that formula (I) compounds stimulate the regeneration of neuronal tissue.

Therefore, as was mentioned above, the present invention relates to the use of a formula (I) compound, or a pharmaceutically acceptable salt, prodrug or solvate thereof, in the preparation of a medicament for regenerating damaged neuronal tissue.

As used herein, "neurogenesis" refers to the proliferation, differentiation, migration or survival of neural cells *in vitro* or *in vivo.* In a particular embodiment, said neural cell is an embryonic, foetal or adult neural stem cell or a progenitor cell. Neurogenesis also refers to a net increase in the number of cells or in cell survival. A "neurogenic agent", as used herein, refers to an agent that can promote neurogenesis.

As used herein, "neuronal tissue" or "nerve tissue" refer to any tissue that includes neuronal cells or neural cells or neurons.

According to a preferred embodiment, said tissue is nerve tissue of the Central Nervous System (CNS).

According to a preferred embodiment, said tissue is nerve tissue of the Peripheral Nervous System.

According to a preferred embodiment, the formula (I) compound used presents formula (II): 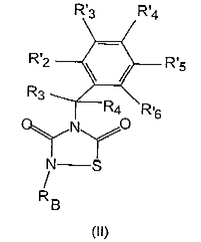 wherein:
R_{B} is selected from substituted or unsubstituted alkyl, substituted or unsubstituted cycloalkyl, haloalkyl, substituted or unsubstituted aryl, substituted or unsubstituted heteroaryl, -OR⁵ and -S(O)ₜ-R⁷;
R³, R⁴, R²', R³', R⁴', R⁵' and R⁶' are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, COR⁷, -C(O)OR⁷, -C(O)NR⁷R⁸ -C=NR⁷, -CN, -OR⁷, OC(O)R⁷, -S(O)ₜ-R⁷, -N R⁷R⁸, -NR⁷C(O) R⁸, -NO₂, -N=CR⁷R⁸ or halogen, wherein R³ and R⁴ together can form a =O group, and wherein any pair of R³R²' R³R⁶', R⁴R²', R⁴R⁶', R²'R³', R³'R⁴', R⁴'R⁵', R⁵'R⁶' or R⁷R⁸ together can form a cyclic substituent; t is 0, 1, 2, 3
R⁵ is selected from hydrogen, alkyl, aryl and heterocyclyl;
R⁷ and R⁸ are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy and halogen.

According to a preferred embodiment, R_{B} is an organic hydrocarbon residue whose skeleton is made up of 8 to 20 atoms selected from C and O.

According to a preferred embodiment, RB includes an aromatic group.

According to a preferred embodiment, R_{B} includes an aryl group whose skeleton is made up of 8 to 20 atoms selected from C and O.

According to a preferred embodiment, R_{B} includes an alkylaryl group whose skeleton is made up of 8 to 20 atoms selected from C and O.

According to a preferred embodiment, R_{B} includes an aralkyl group whose skeleton is made up of 8 Lo 20 atoms selected from C and O.

According to a preferred embodiment, R_{B} has at least 10 aromatic carbons.

According to a preferred embodiment, said aromatic group is directly linked to the N of thiadiazolidine.

According to a preferred embodiment, R_{B} is a substituted or unsubstituted naphthyl group.

According to a preferred embodiment, R_{B} is an unsubstituted alpha-naphthyl group.

According to a preferred embodiment, R_{B} is a group selected from:

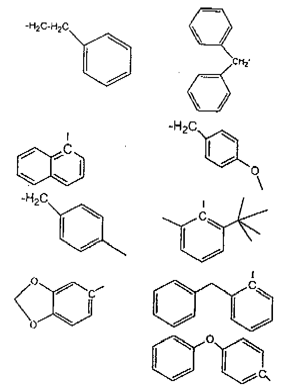

According to a preferred embodiment, R and R⁴ are H.

According to a preferred embodiment, R²', R³', R⁴', R⁵' and R⁶' are each independently selected from hydrogen, substituted or unsubstituted alkyl, -COR⁷, -C(O)OR⁷, -OR⁷, NR⁷R⁸ or halogen, wherein R⁷ and R⁸ are defined as in claim 2.

According to a preferred embodiment, R²', R³', R⁴', R⁵' and R⁶' are H.

According to a preferred embodiment, the formula II compound presents the structure:

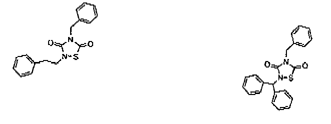

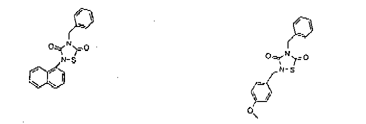

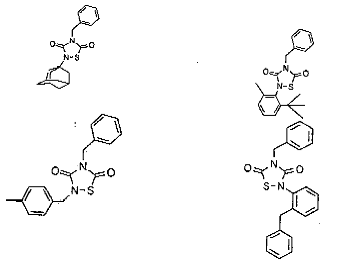

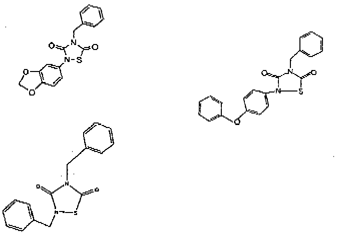

In the above definition of formula (I) compounds these terms have the following meanings:

"Alkyl" refers to straight or branched hydrocarbon chain radicals that consist of carbon and hydrogen atoms, containing no unsaturation and having one to eight carbon atoms, and which are attached to the rest of the molecule by a single bond, e.g. methyl, ethyl, n-propyl, i-propyl, n-butyl, t-butyl, n-pentyl, etc. Alkyl radicals can optionally be substituted by one or more substituents such as an aryl, halogen, hydroxyl, alkoxyl, carboxyl, cyano, carbonyl, acyl, alkoxycarbonyl, amino, nitro, mercapto, alkylthio, etc. If they are substituted by aryl, we have an "Aralkyl" radical, such as benzyl and phenethyl.

"Alkenyl" refers to an alkyl radical with at least 2 C atoms and with one or more unsaturated bonds.

"Cycloalkyl" refers to a stable 3- to 10-membered monocyclic or bicyclic radical, which is saturated or partially saturated, and which only consists of carbon and hydrogen atoms, such as cyclohexyl or adamantyl. Unless specifically stated otherwise in the specifications, the term "cycloalkyl" means that it includes cycloalkyl radicals that are optionally substituted by one or more substituents such as alkyl, halogen, hydroxyl, amino, cyano, nitro, alkoxyl, carboxyl, alkoxycarbonyl, etc.

"Aryl" refers to single-ring and multiple-ring radicals that include multiple-ring radicals containing separate and/or fused aryl groups. Typical aryl groups contain 1 to 3 separate or fused rings from 6 to approximately 18 carbon ring atoms, such as phenyl, naphthyl, indenyl, fenanthryl or anthracyl radicals. The aryl radical can optionally be substituted by one or more substituents such as hydroxyl, mercapto, halogen, alkyl, phenyl, alkoxyl, haloalkyl, nitro, cyano, dialkylamino, aminoalkyl, acyl, alkoxycarbonyl, etc. If they are substituted by alkyl we have an "alkylaryl" radical, such as alkylbenzene or alkylnaphthyl.

"Organic hydrocarbon residue" refers to radicals whose skeleton is exclusively made up of carbon and oxygen atoms. This does not exclude the presence of hydrogen atoms in the chain and other substituents such as halogens, alkenyl, alkyl, cicloalkyl, aryl, alkoxyl, alkoxycarbonyl, aralkyl or alkylaryl.

Said "organic hydrocarbon residues" are preferably selected from aryl, alkyl, aralkyl or alkylaryl radicals.

"Heterocyclyl" refers to a stable 3- to 15-membered ring that consists of carbon atoms and one to five heteroatoms selected from the group consisting of nitrogen, oxygen, and sulphur, preferably a 4- to 8-membered ring with one or more heteroatoms, and more preferably, a 5- or 6-membered ring with one or more heteroatoms. For the purposes of this invention, the heterocycle can be a monocyclic, bicyclic or tricyclic ring system, which can include fused ring systems, and the nitrogen, carbon or sulphur atom in the heterocyclyl radical can optionally be oxidised; the nitrogen atom can optionally be quaternised; and the heterocyclyl radical can be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran, coumarin, morpholine; pyrrole, pyrazole, oxazole, isoxazole, triazole, imidazole, etc.

"Alkoxyl" refers to a radical of the formula -ORa wherein Ra is an alkyl radical as defined above, e.g. methoxyl, ethoxyl, propoxyl, etc.

"Alkoxycarbonyl" refers to a radical of the formula -C(O)ORa wherein Ra is an alkyl radical as defined above, e.g. methoxycarbonyl, ethoxycarbonyl, propoxycarbonyl, etc.

"Alkylthio" refers to a radical of the formula -SRa wherein Ra is an alkyl radical as defined above, e.g. methylthio, ethylthio, propylthio, etc.

"Amino" refers to a radical of the formula -NH₂, -NHRa or -NRaRb, which can optionally be quaternised.

"Halogen" or "halo" refers to bromine, chlorine, iodine or fluorine.

The references herein to groups substituted in the compounds of the present invention refer to the specified group that can be substituted in one or more available positions by one or more suitable groups, e.g. halogen such as fluorine, chlorine, bromine and iodine; cyano; hydroxyl; nitro; azido; alkanoyl such as a C1-6 alkanoyl group such as acyl and suchlike; carboxamide; alkyl groups including groups with 1 to approximately 12 carbon atoms or 1 to approximately 6 carbon atoms, and more preferably, 1 - 3 carbon atoms; alkenyl and alkynyl groups including groups with one of more unsaturated bonds and 2 to approximately 12 carbon atoms or 2 to approximately 6 carbon atoms; alkoxyl groups with one or more bonds to oxygen and 1 to approximately 12 carbon atoms or 1 to approximately 6 carbons atoms; aryloxyl such as phenoxyl; alkylthio groups including groups with one or more thioether bonds and 1 to approximately 12 carbon atoms or 1 to approximately 6 carbon atoms; alkylsulphinyl groups including groups with one or more sulphinyl bonds and 1 to approximately 12 carbon atoms or 1 to approximately 6 carbon atoms; alkylsulphonyl groups including groups with one or more sulphonyl bonds and 1 to approximately 12 carbon atoms or 1 to approximately 6 carbon atoms; aminoalkyl groups such as groups with one or more N atoms and 1 to approximately 12 carbon atoms or 1 to approximately 6 carbon atoms; carbocyclic aryl with 6 or more carbon atoms, particularly phenyl or naphthyl and aralkyl such as benzyl. Unless otherwise stated, an optionally substituted group can have a substituent in each substitutable position of the group, and each substitution is independent of the other.

Particular individual compounds of the invention include the compounds defined in the claims and examples.

Unless otherwise stated, the compounds of the invention also include compounds that only differ in terms of the presence of one or more isotopically enriched atoms. For example, compounds with these structures, except for the substitution of a hydrogen atom by a deuterium or tritium atom or the substitution of a carbon atom by a ¹³C or ¹⁴C enriched carbon atom or a ¹⁵N enriched nitrogen atom, are within the scope of this invention.

The term "salts, solvates, pharmaceutically acceptable prodrugs" refers to any salt, ester, pharmaceutically acceptable solvate, or any other compound that, when administered to a recipient, is capable of providing (directly or indirectly) a compound as described herein. However, it should be appreciated that salts that are not pharmaceutically acceptable also lie within the scope of the invention, as they can be used in the preparation of pharmaceutically acceptable salts. The preparation of salts, prodrugs and derivatives can be carried out using known methods.

For instance, pharmaceutically acceptable salts of compounds anticipated herein are synthesised by conventional chemical methods using a parent compound containing a base or an acid residue. Generally, such salts are prepared, for example, by making free acid or base forms of the compounds react with a stoichiometric quantity of the appropriate base or acid in water or in an organic solvent, or in a mixture of the two. Generally, non-aqueous media like ether, ethyl acetate, ethanol, isopropanol or acetonitrile are preferred. Examples of acid addition salts include mineral acid addition salts such as hydrochloride, hydrobromide, hydroiodide, sulphate, nitrate, phosphate, and organic acid addition salts such as acetate, maleate, fumarate, citrate, oxalate, succinate, tartrate, malate, mandelate, methanesulphonate and p-toluenesulphonate. Examples of base addition salts include inorganic salts such as sodium, potassium, calcium, ammonium, magnesium, aluminium and lithium salts, and organic base salts such as ethylenediamine, ethanolamine, NN-dialkyl-ethanolamine, triethanolamine, glucamine and basic amino acid salts.

Particularly favoured derivatives or prodrugs are those that increase the bioavailability of the compounds of this invention when such compounds are administered to a patient (e.g. by making an orally administered compound more easily absorbed by the blood), or which improve the release of the parent compound in a biological compartment (e.g. the brain or lymphatic system) that is related to the parent species.

Any compound that is a prodrug of a formula (I) compound is within the scope of the invention. The term "prodrug" is used in its broadest sense and covers derivatives that are converted in vivo to the compounds of the invention. Such derivatives will be well known by persons skilled in the art, and include, depending on the functional groups present in the molecule and without limitation, the following derivatives of the present compounds: esters, amino acid esters, phosphate esters, sulphonate esters of metal salts, carbamates and amides.

Formula (I) compounds can be in crystalline form either as free compounds or as solvates and it is intended that both forms should be within the scope of the present invention. Methods of solvation are generally known within the art. Suitable solvates are pharmaceutically acceptable solvates. In a particular embodiment, the solvate is a hydrate.

Formula (I) compounds or their salts or solvates are preferably in a pharmaceutically acceptable or substantially pure form. Pharmaceutically acceptable form means, among other factors, that they have a pharmaceutically acceptable level of purity excluding normal pharmaceutical additives such as diluents and carriers, and not including any material considered toxic at normal dosage levels. Levels of purity for the active ingredient are preferably higher than 50%, more preferably higher than 70% and most preferably higher than 90%. In a preferred embodiment, they are higher than 95% of the formula (I) compound, or of its salts, solvates or prodrugs.

The formula (I) compounds defined above can be obtained using available synthetic procedures that are disclosed, for example, in applications WO 0185685, US2003/0195238 and EP04075997.9.

Formula (I) compounds, their pharmaceutically acceptable salts, prodrugs or solvates thereof, can therefore be used in the prevention and/or treatment of a disease or condition that requires the regeneration of neuronal tissue. Pharmaceutical compositions containing a therapeutically effective quantity of a formula (I) compound, its pharmaceutically acceptable salts, prodrugs or solvate thereof, together with pharmaceutically acceptable excipients, are an additional aspect of the present invention.

The therapeutically effective quantity of formula (I) compound, its pharmaceutically acceptable salts, prodrugs or solvates thereof that must be administered and the dosage for treating a pathological state with said compounds will depend on numerous factors, including age, the state of the patient, the severity of the disease, the route and frequency of administration, the modulator compound to be used, etc.

The compounds and compositions of this invention can be used alone or in conjunction with other drugs to provide a combined therapy. Other drugs can form part of the same composition or be provided as a separate composition for administration at the same time or in a different moment.

A combined therapy can be particularly useful due to the type of pathologies to be treated with these compounds as defined in the present invention; these pathologies are especially complex, as patients generally present a combination of symptoms and a variety of injuries or disorders. It can therefore be useful to combine several drugs, each of which is specifically aimed at preventing, relieving or curing a particular symptom, injury or disorder, or even several of them, resulting in a combined therapy directed at the disease or condition as a whole, taking into account many, most or all of the aspects involved therein.

The drugs to be combined with the compounds of the present invention can be drugs approved for the treatment of a disease, or newly developed drugs.

According to a preferred embodiment, formula I compounds are used to produce a medicament for regenerating neuronal tissue in pathologies or conditions that involve neuronal damage or death in the central nervous system or peripheral nervous system.

Neuronal death can be placed in two broad groups:
necrosis and apoptosis.

According to a preferred embodiment, formula I compounds are used to produce a medicament for regenerating neuronal tissue in pathologies or conditions that involve neuronal damage or death caused by necrosis in the central nervous system or peripheral nervous system.

According to a preferred embodiment, formula I compounds are used to produce a medicament for regenerating neuronal tissue in pathologies or conditions that involve neuronal damage or death caused by apoptosis in the central nervous system or peripheral nervous system.

According to a preferred embodiment, formula I compounds are used to produce a medicament for regenerating neuronal tissue damaged by neuronal damage or death that occur in patients with mood disorders, such as depression or bipolar disorder, or with an acute attention deficit disorder.

According to a preferred embodiment, formula I compounds are used to produce a medicament for regenerating neuronal tissue damaged by neuronal damage or death that occur in patients with a chronic neurodegenerative disease, such as Alzheimer's disease, Parkinson's disease or multiple sclerosis.

According to a preferred embodiment, formula I compounds are used to produce a medicament for regenerating neuronal tissue damaged by neuronal damage or death caused by acute neuronal injury, such as crush injury, acute stroke, ischaemia, neurotraumatic insult, neurotrauma or spinal cord injury.

According to a preferred embodiment, formula I compounds are used to produce a medicament for regenerating neuronal tissue damaged by neuronal damage or death that occurs in cases of stroke; epilepsy; prion diseases, such as Creutzfeld-Jakob disease and Gerstmann-Straussler-Scheinker syndrome; Huntington's disease; acquired immune deficiency syndrome; states of muscular dystrophy; amyotrophic lateral sclerosis; diabetic neuropathy, frontotemporal dementia (Pick's disease).

According to a preferred embodiment, formula I compounds are used to produce a medicament for regenerating neuronal tissue damaged by neuronal damage or death in optic nerve injury, such as that caused by glaucoma.

According to a preferred embodiment, formula I compounds are used to produce a medicament for regenerating neuronal tissue damaged by neuronal damage or death caused by acute neuronal injury, such as crush injury, neurotraumatic insult, neurotrauma or spinal cord injury.

According to a preferred embodiment, formula I compounds are used to produce a medicament for regenerating neuronal tissue damaged by neuronal damage or death caused by prion diseases, such as Creutzfeld-Jakob disease, Gerstmann-Sträussler-Scheinker syndrome or diabetic neuropathy.

According to another essential aspect, the present invention relates to a method for regenerating neuronal tissue in a patient who presents at least one pathology that involves neuronal damage or death in the central nervous system or peripheral nervous system that comprises the administration of a pharmaceutically acceptable quantity of a general formula (I) compound: 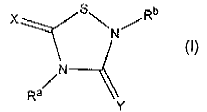 or a pharmaceutically acceptable salt, prodrug or solvate thereof, wherein:
- R^{a} and R^{b}: are each independently selected from hydrogen, substituted or unsubstituted alkyl, substituted or unsubstituted cycloalkyl, haloalkyl, substituted or unsubstituted aryl, substituted or unsubstituted -(Z)ₙ-aryl, substituted or unsubstituted heteroaryl, -OR⁵, -C(O)R⁵, -C(O)OR⁵, -(Z)ₙC(O)OR⁵ and -S(O)ₜ-R⁷;
- Z: is independently selected from -C(R³) (R⁴)-, -C(O)-, -O-, -C(=NR⁵)-, -S(O)ₜ-R⁷ and N(R⁵)-;
- n: is zero, one or two;
- t: is zero, one or two;
- R³ and R⁴: are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, COR⁷, -C(O)OR⁷, -C(O)NR⁷R⁸ -C=NR⁷, -CN, -OR⁷, -OC(O)R⁷, -S(O)ₜ-R⁷, -NR⁷R⁸, -NR⁷C(O)R⁸, -NO₂, -N=CR⁷R⁸ or halogen, wherein R³ and R⁴ together can form a =O group;
- X and Y: are each independently selected from =O, =S, =N(R⁵) and =C(R¹) (R²) ;
- R¹ and R²: are each independently selected from hydrogen, substituted or unsubstituted alkyl and substituted or unsubstituted cycloalkyl;
- R⁵: is selected from hydrogen, substituted or unsubstituted alkyl, substituted or unsubstituted aryl and substituted or unsubstituted heterocyclyl;
- R⁷ and R⁸: are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy and halogen.

The following examples are provided purely as an additional illustration of the invention and must not be taken as a definition of the limits of the invention.

### EXAMPLES:

Neurospheres of the cerebral cortex and hippocampus of 2-day-old postnatal rats were grown in suspension culture for 7 days in the presence or absence of a formula (I) compound according to the invention, after which they were transferred to slides covered with polylysine and 24 h later (once adhered) immunofluorescence analyses were performed with anti-GFAP antibodies to view the astrocyte differentiation, or with anti-MAP2 to view neuronal differentiation. The images were captured on a TCS SP2 confocal microscope (Leica Microsystems).

### Example 1:

The aforementioned test was carried out using as formula (I) compound the compound 2,4-dibenzyl-[1,2,4]thiadiazolidine-3,5-dione[1].

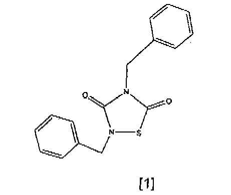

The results obtained show that said compound [1] induces astrocyte (Figure 1) and neuron (Figure 2) differentiation of cells present in isolated neurospheres of 2-day-old neonatal rats. In said figures "basal" refers to neurospheres grown in the absence of the compound (1); "x" indicates the number of magnifications; "DAPI" refers to fluorescent staining by 4',6-diamidino-2-phenylindole (DAPI).

### Example 2:

The test was carried out using as formula (I) compound the compound 4-benzyl-2-naphthalene-1-yl-[1,2,4]thiadiazolidine-3,5-dione[2].

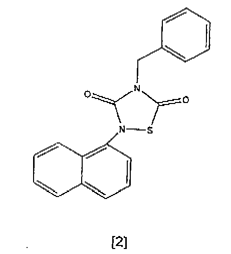

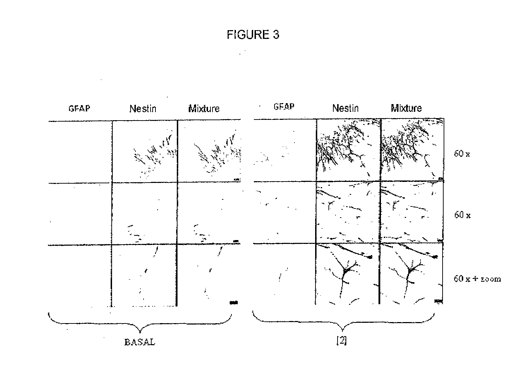

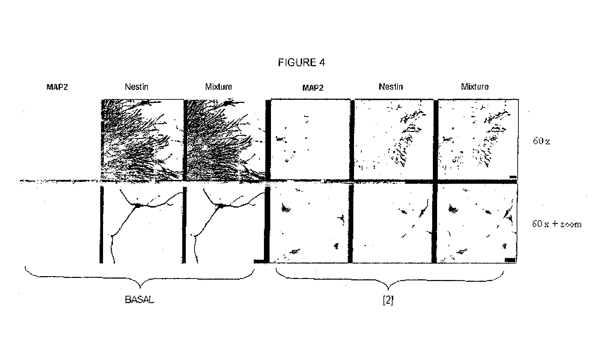

The results obtained show that said compound [2] induces astrocyte (see Figure 3) and neuron (see Figure 4) differentiation of cells present in isolated neurospheres of 2-day-old neonatal rats. In said figures "basal" refers to neurospheres grown in the absence of the compound of formula (I); "x" indicates the number of magnifications; GFAP refers to viewing by GFAP (glial fibrillary acidic protein) staining; "Nestin" refers to staining by nestin, which is present in immature neural cells; MAP-2 refers to viewing by fluorescent staining by MAP-2 (microtubule-associated protein 2); "Mixture" refers to staining by both nestin and GFAP in the case of Figure 3, and by nestin and MAP-2 in the case of Figure 4.

The figures also show a migration of the differentiated cells: in Figure 3, the upper row of photographs has been taken magnifying cells in the neurosphere 60-fold; the central row of photographs has been taken using the same magnification, but for cells that have migrated out of the neurosphere; the lower row also shows photographs of cells outside the neurosphere, magnifying cells in the neurosphere 60-fold plus zoom.

In Figure 4, the upper row of photographs has been taken magnifying cells in the neurosphere 60-fold; the lower row in the case of the "basal" shows photographs of cells that have migrated out of the neurosphere, magnifying the image 60-fold plus zoom. In the case of compound [2], the lower row shows photographs magnifying cells in the neurosphere 60-fold plus zoom.

### Example 3:

The test was carried out using as formula (I) compound the compound 2-Benzhydryl-4-benzyl-[1,2,4]thiadiazolidine-3,5-dione[3].

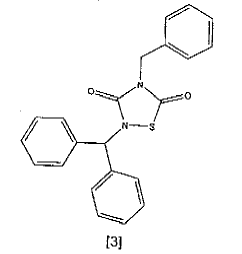

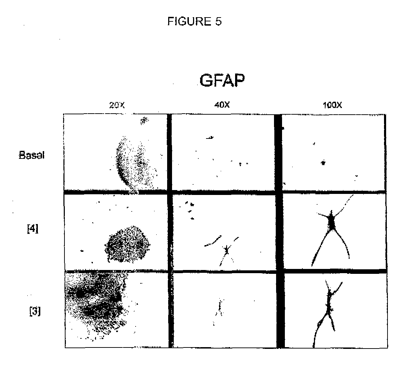

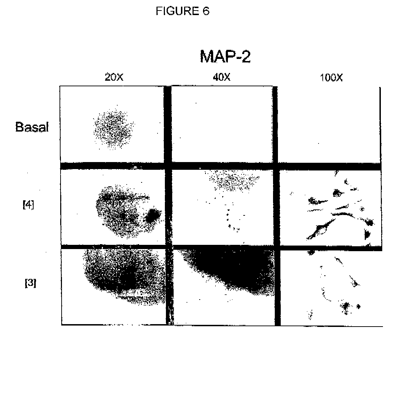

The results obtained show that said compound [3] induces astrocyte (Figure 5) and neuronal (Figure 6) differentiation of cells present in isolated neurospheres of 2-day-old neonatal rats. In said figures "basal" refers to neurospheres grown in the absence of the compound (I); "x" indicates the number of magnifications; GFAP in Figure 5 refers to viewing by GFAP (glial fibrillary acidic protein) staining; MAP-2 in Figure 6 refers to viewing by fluorescent staining by MAF-2 (microtubule-associated protein 2).

### Example 4:

The test was carried out using as formula (I) compound the compound 4-benzyl-2-(4-phenoxy-phenyl)-[1,2,4]thiadiazolidine-3,5-dione[4].

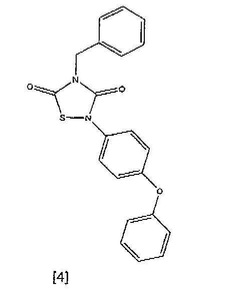

The results obtained show that said compound [4] induces astrocyte (Figure 5) and neuronal (Figure 6) differentiation of cells present in isolated neurospheres of 2-day-old neonatal rats. In said figures "basal" refers to neurospheres grown in the absence of the compound (I); "x" indicates the number of magnifications; GFAP in Figure 5 refers to viewing by GFAP (glial fibrillary acidic protein) staining; MAP-2 in Figure 6 refers to viewing by fluorescent staining by MAP-2 (microtubule-associated protein 2).

## Claims

1. Use of a general formula (I) compound: 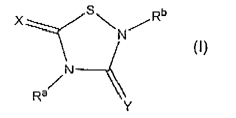 or a pharmaceutically acceptable salt, prodrug or solvate thereof, wherein:
R^{a} and R^{b} are each independently selected from hydrogen, substituted or unsubstituted alkyl, substituted or unsubstituted cycloalkyl, haloalkyl, substituted or unsubstituted aryl, substituted or unsubstituted -(Z)ₙ-aryl, substituted or unsubstituted heteroaryl, -OR⁵, -C(O)R⁵, -C(O)OR⁵, -(Z)ₙC(O)OR⁵ and -S(O)ₜ-R⁷;
Z is independently selected from -C(R³) (R⁴)-, -C(O)-, -O-, -C(=NR⁵)-, -S(O)ₜ-R⁷ and N(R⁵)-;
n is zero, one or two;
t is zero, one or two;
R³ and R⁴ are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, COR⁷, -C(O)OR⁷, -C(O)NR⁷R⁸ -C=NR⁷, -CN, -OR⁷, -OC(O)R⁷, -S(O)ₜ-R⁷, -NR⁷R⁸, -NR⁷C(O)R⁸, -NO₂, -N=CR⁷R⁸ or halogen, wherein R³ and R⁴ together can form a =O group;
X and Y are each independently selected from =O, =S, =N(R⁵) and =C(R¹) (R²);
R¹ and R² are each independently selected from hydrogen, substituted or unsubstituted alkyl and substituted or unsubstituted cycloalkyl;
R⁵ is selected from hydrogen, substituted or unsubstituted alkyl, substituted or unsubstituted aryl and substituted or unsubstituted heterocyclyl;
R⁷ and R⁸ are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy and halogen;
in the preparation of a medicament for regenerating neuronal tissue damaged by neuronal damage or death that occur in patients with mood disorders, such as depression or bipolar disorder, with an acute attention deficit disorder; with acute neuronal injury, such as crush injury, acute stroke, ischaemia, neurotraumatic insult, neurotrauma or spinal cord injury; with optic nerve injury, such as that caused by glaucoma; with prion diseases, such as Creutzfeld-Jakob disease and Gerstmann-Sträussler-Scheinker syndrome; with states of muscular dystrophy; or with diabetic neuropathy.

2. Use according to claim 1, wherein the formula (I) compound that is used presents formula (II): 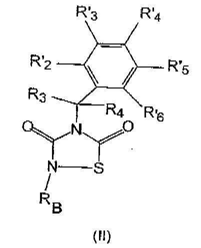 wherein:
R_{B} is selected from substituted or unsubstituted alkyl, substituted or unsubstituted cycloalkyl, haloalkyl, substituted or unsubstituted aryl, substituted or unsubstituted aralkyl, substituted or unsubstituted alkylaryl, substituted or unsubstituted heteroaryl, -OR⁵ and -S(O)ₜ-R⁷;
R³, R⁴, R²', R³', R⁴', R⁵', and R⁶' are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, COR⁷, -C(O)OR⁷, -C(O)NR⁷R⁸ -C=NR⁷, -CN, -OR⁷, OC(O)R⁷, -S(O)ₜ-R⁷, -N R⁷R⁸, -NR⁷C(O)R⁸, -NO₂,-N=CR⁷R⁸ or halogen, wherein R³ and R⁴ together can form a =O group, and wherein any pair of R³R²', R³R⁶', R⁴R²', R⁴R⁶', R²'R³', R³'R⁴', R⁴'R⁵', R⁵'R⁶' or R⁷R⁸ together can form a cyclic substituent; t is 0, 1, 2, 3
R⁵ is selected from hydrogen, alkyl, aryl and heterocyclyl;
R⁷ and R⁸ are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy and halogen.

3. Use according to claim 2, wherein R_{B} is an organic hydrocarbon residue whose skeleton is made up of 8 to 20 atoms selected from C and 0.

4. Use according to claims 2 and 3, wherein R_{B} includes an aromatic group.

5. Use according to claims 2 and 3, wherein R_{B} has at least 10 aromatic carbons.

6. Use according to claims 1 to 3, wherein the aromatic group is directly linked to the N of thiadiazolidine.

7. Use according to claim 5, wherein R_{B} is a substituted or unsubstituted naphthyl group.

8. Use according to claim 7, wherein R_{B} is an unsubstituted alpha-naphthyl group.

9. Use according to claim 1, wherein R_{B} is a group selected from:

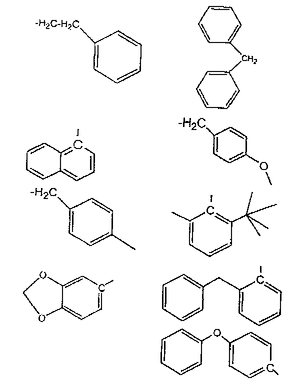

10. Use according to claim 2, wherein R³ and R⁴ are H.

11. Use according to claim 2, wherein R²', R³', R⁴', R⁵' and R⁶' are each independently selected from hydrogen, substituted or unsubstituted alkyl, -COR⁷, -C(O)OR⁷, -OR⁷, NR⁷R⁸ or halogen, wherein R⁷ and R⁸ are defined as in claim 2.

12. Use according to claim 2, wherein R²', R³', R⁴', R⁵' and R⁶' are H.

13. Use according to claim 2, wherein the formula II compound presents the structure:

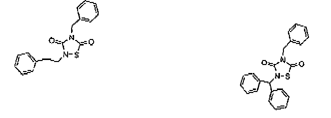

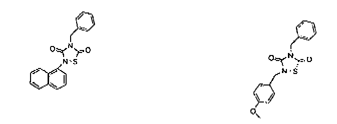

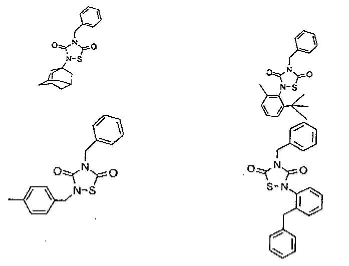

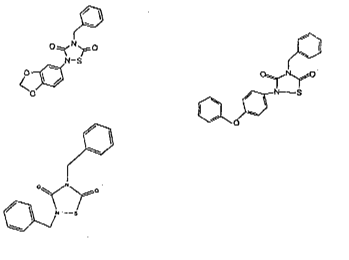

14. Use according to claim 1, wherein the medicament is for regenerating neuronal tissue damaged by neuronal damage or death that occur in patients with mood disorders, such as depression or bipolar disorder, or with an acute attention deficit disorder.

15. Use according to claim 1, wherein the medicament is for regenerating neuronal tissue damaged by neuronal damage or death caused by acute neuronal injury, such as crush injury, acute stroke, ischaemia, neurotraumatic insult, neurotrauma or spinal cord injury.

16. Use according to claim 1, wherein the medicament is for regenerating neuronal tissue damaged by neuronal damage or death that occurs in cases of prion diseases, such as Creutzfeld-Jakob disease and Gerstmann-Sträussler-Scheinker syndrome; states of muscular dystrophy; diabetic neuropathy.

17. Use according to claim 1, wherein the medicament is for regenerating neuronal tissue damaged by neuronal damage or death in optic nerve injury, such as that caused by glaucoma.

18. Method for regenerating neuronal tissue in a patient who presents at least one pathology that involves neuronal damage or death in the central nervous system or peripheral nervous system that comprises the administration of a pharmaceutically acceptable quantity of a general formula (I) compound: 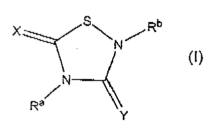 or a pharmaceutically acceptable salt, prodrug or solvate thereof, wherein:
R^{a} and R^{b} are each independently selected from hydrogen, substituted or unsubstituted alkyl, substituted or unsubstituted cycloalkyl, haloalkyl, substituted or unsubstituted aryl, substituted or unsubstituted -(Z)ₙ-aryl, substituted or unsubstituted heteroaryl, -OR⁵, -C(O)R⁵, -C(O)OR⁵, -(Z)ₙC(O)OR⁵ and -S(O)ₜ-R⁷;
Z is independently selected from -C(R³) (R⁴)-, -C(O)-, -O-, -C(=NR⁵)-, -S(O)ₜ-R⁷ and N(R⁵)-;
n is zero, one or two;
t is zero, one or two;
R³ and R⁴ are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, COR⁷, -C(O)OR⁷, -C(O)NR⁷R⁸ -C=NR⁷, -CN, -OR⁷, -OC(O)R⁷, -S(O)ₜ-R⁷, -NR⁷R⁸, -NR⁷C(O)R⁸, -NO₂, -N=CR⁷R⁸ or halogen, wherein R³ and R⁴ together can form a =O group;
X and Y are each independently selected from =O, =S, =N(R⁵) and =C(R¹) (R²) ;
R¹ and R² are each independently selected from hydrogen, substituted or unsubstituted alkyl and substituted or unsubstituted cycloalkyl;
R⁵ is selected from hydrogen, substituted or unsubstituted alkyl, substituted or unsubstituted aryl and substituted or unsubstituted heterocyclyl;
R⁷ and R⁸ are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy and halogen.
